# EUROPÄISCHE PATENTSCHRIFT

(11) **EP 0 953 329 B1**
(45) Veröffentlichungstag und Bekanntmachung des Hinweises auf die Patenterteilung: **23.06.2004**
(21) Anmeldenummer: 99107805.6
(22) Anmeldetag: 20.04.1999
(51) Int. Cl.: A61K 6/04

(54) **Dentallegierungen auf Basis von Gold-Palladium-Silber**
Gold-palladium-silver based dental alloys
Alliages dentaires à base d' or-palladium-argent

(30) Priorität: 30.04.1998 DE 19819369
(43) Veröffentlichungstag der Anmeldung: 03.11.1999
(73) Patentinhaber: DeguDent GmbH, 63457 Hanau (DE)
(72) Erfinder: Kempf, Bernd Dr., 63839 Kleinwallstadt (DE); Schöck, Gernot, 63486 Bruchköbel (DE); Hathaway, Doris, 63457 Hanau (DE); Ringelstein, Hans-Martin, 60385 Frankfurt/M (DE); Rothaut, Josef Dr., 63755 Alzenau (DE); Völkl, Lothar Dr., 63773 Goldbach (DE)
(74) Vertreter: Stoffregen, Hans-Herbert, Dr. Dipl.-Phys.

(56) Entgegenhaltungen:
- DE-A- 2 440 425
- DE-A- 2 942 373
- DE-A- 3 146 794
- DE-B- 2 828 304
- DE-U- 8 705 058

## Beschreibung

Die Erfindung betrifft eine Dentallegierung auf Basis von Gold-Palladium-Silber zur Herstellung von festsitzendem oder herausnehmbarem, mit Dentalkeramik verblendbarem Zahnersatz.

Festsitzender und herausnehmbarer Zahnersatz wird häufig aus korrosionsbeständigen biokompatiblen Edelmetalllegierungen hergestellt, wobei das gegossene Objekt anschließend oft mit Dentalkeramik verblendet wird, um ein dem natürlichen Zahn entsprechendes Aussehen zu erzielen. Die Eignung von Legierungen für diesen Zweck ist an eine Reihe von Eigenschaften geknüpft, die auf die Dentalkeramik abgestimmt sein müssen, wie thermischer Ausdehnungskoeffizient, Schmelzintervall und Haftung zwischen Keramik und Legierung. Grundvoraussetzung ist auch eine gute Korrosionsbeständigkeit und eine ausreichende Festigkeit, um den Belastungen beim Kauvorgang zu widerstehen. Nach ihrer mechanischen Belastbarkeit werden Dentallegierungen in verschiedene Klassen von Typ 1 bis Typ 4 eingeteilt (EN ISO 8891). Die höchste Festigkeit und damit die breiteste Indikation besitzen Typ 4-Legierungen. Hier wird eine Mindestdehngrenze von 450 MPa gefordert. Die in früheren Normenausgaben auch vorgegebenen Härtewerte sind in der neuen Ausgabe der Norm nicht mehr vorgesehen. Trotzdem wird im Markt bis heute auch die Angabe der Härtewerte praktiziert. Durch das einfache Meßverfahren und die enge Verknüpfung mit der mechanischen Festigkeit wird die Härtemessung in der Legierungsentwicklung zur ersten Charakterisierung stets eingesetzt. Erfahrungsgemäß lassen sich mechanische Festigkeiten von 450 MPa ab Härtewerten von ca. 180 - 200 HV5 erreichen. Für verblendbare Legierungen (Metall-Keramiksysteme) erfolgt die Charakterisierung nach EN-ISO 9693. Danach sind Mindestfestigkeiten von 250 MPa gefordert. Im Markt wird allerdings bis heute in der Regel auch für verblendbare Legierungen die Einteilung in die Typen 1-4 bevorzugt.

Aus wirtschaftlichen Gründen werden seit vielen Jahren neben den hochgoldhaltigen Edelmetallegierungen auch goldreduzierte Legierungen eingesetzt. Hier kann grob zwischen Gold-Palladium-, Gold-Palladium-Silber und Palladium-Silber-Gold-Legierungen unterschieden werden. Die Reihenfolge der Elemente entspricht dabei dem relativen Mengenanteil. Auch diese Dentallegierungen haben sich seit vielen Jahren als Zahnersatzmaterialien bewährt, auch wenn in der Regel nicht ganz so gute Korrosionsbeständigkeiten erreichbar sind, wie bei den hochgoldhaltigen Legierungen.

Die zahlreichen, oben schon erwähnten Anforderungen, die an diese Legierungen gestellt werden, konnten bisher nur mit in aller Regel sehr kompliziert aufgebauten Legierungssystemen erfüllt werden.

Neben den bereits erwähnten Edelmetallen enthalten diese Legierungen zur Erhöhung der Härte und der mechanischen Festigkeit eine ganze Reihe verschiedener Nichtedelmetalle zulegiert. Weitere Elemente werden zulegiert, um die Feinabstimmung weiterer zahntechnisch relevanter Daten wie thermischer Ausdehnungskoeffizient, Keramikhaftung, Oxidfarbe oder ausreichende Duktilität bei hoher Temperatur zu gewährleisten. Gebräuchliche Legierungselemente sind Kupfer, Indium, Zink, Zinn, Eisen, Tantal, Gallium u.a. Nur im Bereich der hochgoldhaltigen Aufbrennlegierungen sind bisher Legierungen bekannt, die lediglich mit einem einzigen Nichtedelmetall, und zwar mit Zinn, alle diese komplexen Anforderungen erfüllen können und sich außerdem durch eine hohe Korrosionsbeständigkeit auszeichnen.

Im Bereich der goldreduzierten Gold-Palladium-Silberlegierungen ist es aber bisher offenbar notwendig, mehrere Nichtedelmetalle den Hauptlegierungselementen Gold, Palladium und Silber zuzulegieren, um alle für Zahnersatz notwendigen Eigenschaften zu erreichen. Bekannte goldreduzierte Dentallegierungen enthalten in der Regel zwei oder mehr Unedelmetallegierungselemente.

Die Patentschrift FR 2620133 sieht wahlweise Zinn- und/oder Indium als Nichtedelmetall vor. Darüberhinaus ist ein Mindestgehalt von 5 Gew%. Pt vorgesehen. Es ist bekannt, daß es bei gleichzeitiger Anwesenheit von Pt- und Sn in Gold-Pd-Ag-Legierungen zur Ausbildung von groben Pt-Sn-Ausscheidungen kommt, die sowohl die Duktilität als auch die Festigkeit negativ beeinflussen.

In der Patentschrift DE 24 40 425 werden goldarme Edelmetall-Legierungen beschrieben. Der Zinngehalt ist hier generell auf maximal 6 Gew.% beschränkt. Die Legierungen können optional die Nichtedelmetalle Cu, Fe, Re, Sn, In und Zn enthalten. Weiterhin ergibt sich, daß für Legierungen mit merklichen Goldgehalten (ab 22 Gew%) neben Zinn noch zwingend Kupfer und Eisen benötigt werden.

In DE 31 46 794 A1 werden goldreduzierte Edelmetallegierungen beschrieben, die neben Gallium mindestens zwei weitere Nichtedelmetalle zwingend enthalten.

In DE 32 23 061 A1 werden goldreduzierte Legierungen mit einem Goldgehalt von maximal 35 Gew% beschrieben, die neben einem In-Gehalt von mindestens 7 Gew.% zusätzlich Sn und Zn enthalten können. Die angeführten Beispiele enthalten In und Zn gleichzeitig. Die Korrosionsbeständigkeit dieser Legierungen ist jedoch ungenügend.

In der Auslegeschrift DE 28 28 304 B1 werden goldarme Edelmetall-Legierungen beschrieben, die zwingend Ti-Gehalte von 0.05-0.5% enthalten und optional zusätzlich die Nichtedelmetalle Kupfer, Eisen, Zinn und Indium.

In der Patentschrift DE 29 42 373 C2 werden goldreduzierte Dentallegierungen beschrieben, die zwingend 0.01 - 5% Si, Ge und Bor enthalten und darüberhinaus In und Sn enthalten können.

Im Zuge eines allgemein gestiegenen Gesundheitsbewußtseins und einer generell zu beobachtenden höheren Anfälligkeit des Menschen gegenüber Allergien und Unverträglichkeiten ist auch die Biokompatibilität von Dentallegierungen verstärkt in die Diskussion geraten. Bisherige Untersuchungen zeigen, daß Art und Menge der durch Korrosionsvorgänge in Lösung gehenden Bestandteile einer Legierung für die Biokompatibilität entscheidend sind. Die Ursachen der Korrosion und die möglichen Wirkungen der Korrosionsprodukte auf den Organismus sind dabei sehr komplexer Natur. Untersuchungen deuten darauf hin, daß speziell die während des Keramikbrandes auftretende thermische Belastung und Oxidation der Aufbrennlegierungen ein wesentlicher Faktor ist, der die Korrosionsbeständigkeit der Legierungen herabsetzt. Anzustreben ist generell eine möglichst gute Korrosionsbeständigkeit und eine möglichst geringe Anzahl der Legierungskomponenten, speziell der Nichtedelmetalle, um dadurch die Wahrscheinlichkeit einer allergischen Reaktion auf eine bestimmte Komponente so gering wie möglich zu halten. Selbstverständlich sollten nur Elemente Verwendung finden, die keinerlei toxische Wirkungen besitzen.

Es war daher Aufgabe der Erfindung, preisgünstige, goldreduzierte Edelmetall-Legierungen für mit Keramik verblendbare Dentalgußteile zu entwickeln, die auch zur Erzielung einer für Typ 4-Legierungen geforderten Festigkeit als einziges Unedelmetall das Element Zinn enthalten. Außerdem sollten diese Legierungen eine bessere Korrosionsbeständigkeit aufweisen als die bisherigen Legierungen und alle sonstigen für Aufbrennlegierungen notwendigen Eigenschaften besitzen wie Festigkeit, Härte, Duktilität, Wärmeausdehnungskoeffizient, Keramikhaftung und Hochtemperaturstabilität. Es wurde von vornherein zur Aufgabe gestellt, als Unedelmetall Zinn einzusetzen, da die Unbedenklichkeit des Elementes Zinn, etwa durch seine vielfältige Verwendung in Produkten des täglichen Lebens (als Zinngeschirr oder als Weißblech) oder zur Verpackung von Nahrungsmitteln besonders gut belegt ist.

Diese Aufgabe wird erfindungsgemäß durch eine Dentallegierung auf Basis Gold-Palladium-Silber gelöst, die aus 46-49 Gew.% Gold, 28-35 Gew.% Palladium, 20-23 Gew.% Silber und 3,5-6 Gew.% Zinn besteht, wobei das Verhältnis von Palladium zu Zinn im Bereich 6 bis 8,5 liegt.

Gegenstand der Erfindung ist somit eine wie vorstehend charakterisierte Dentallegierung. Gegenstand der Erfindung ist weiterhin die Verwendung einer derartigen Dentallegierung zur Herstellung von mit Dentalkeramik verblendbarem Zahnersatz.

Gegenstand der Erfindung ist schließlich festsitzender oder herausnehmbarer, mit Dentalkeramik verblendeter Zahnersatz, bei dem das Metallgerüst aus einer derartigen Legierung gefertigt ist.

Überraschenderweise zeigt es sich, daß alle genannten Anforderungen für den Einsatz als Dentalwerkstoff von goldreduzierten Legierungen erfüllt werden können, die als einziges Nichtedelmetall Zinn in diesem exakt spezifizierten Mengenanteil enthalten. Diese Legierungen besitzen eine exzellente Korrosionsbeständigkeit, die deutlich über der Korrosionsbeständigkeit der heute bekannten goldreduzierten Legierungen liegt und weisen damit eine außergewöhnliche Biokompatibilität auf. Die mechanischen Kenndaten erreichen alle Anforderungen an Typ 4-Legierungen, unter der Voraussetzung, daß der Zinngehalt dieser Legierungen in definierter Weise an die Mengenanteile der übrigen Legierungskomponenten Au, Ag und speziell des Pd angepaßt ist. Diese Legierungen besitzen aufgrund der sehr guten Korrosionsbeständigkeit und der Tatsache, daß die Unbedenklichkeit des Nichtedelmetalls Zinn durch seine vielfältige Verwendung in Produkten des täglichen Lebens belegt ist, eine außergewöhnliche Biokompatibilität.

Neben den genannten Legierungsbestandteilen in den definierten Mengenanteilen können die erfindungsgemäßen Dentallegierungen noch 0-1 Gew.% eines hochschmelzenden Elementes wie Ir, Ru, Rh als Kornfeinungsmittel und und/oder 0-2 Gew.% Pt enthalten.

Die erfindungsgemäßen Legierungen besitzen sehr gute Festigkeitswerte und Härten entsprechend Typ 4-Legierungen. Weiterhin zeigen sie eine besonders helle Oxidfarbe. Ihre Korrosionsbeständigkeit ist vorzüglich.

In Tabelle 1 sind eine Reihe von Legierungen nach ihrer Zusammensetzung aufgeführt. In der letzten Spalte ist das jeweilige Verhältnis Pd:Sn angegeben, für die Legierungen, die nur Sn als Nichtedelmetall enthalten. Die Legierungen 1-5 sind Legierungen, die dem Stand der Technik entsprechen. Sie enthalten mindestens zwei Nichtedelmetalle. Die Legierungen 6 und 7 entsprechen der Erfindung.

**Tabelle 1**

| Legierungszusammensetzungen | | | | | | |
|---|---|---|---|---|---|---|
| Legierung | Au | Pd | Ag | Sn | Sonstige | Pd : Sn |
| 1 | 51.1 | 38.5 | - | - | In9 Ga1.2 Ir | |
| 2 | 49,6 | 29 | 17,5 | 3 | Ga0.5 | |
| 3 | 41 | 36 | 18,2 | 3 | Ga1.5 Ir Re | |
| 4 | 43 | 31,8 | 19,5 | 2.5 | In2,5 Re | |
| 5 | 39.1 | 37.5 | 17.5 | 5 | Ga0.5 Ir Re | |
| 6 | 46 | 29,8 | 20 | 4 | Ir | 7.45 |
| 7 | 47.3 | 28.9 | 20 | 3.6 | Ir | 8.03 |

Zur Charakterisierung der Raumtemperaturfestigkeit sind in Tabelle 2 die Härtewerte nach dem Guß (HV-Guß), im ausgehärteten Zustand (HV-hart), und nach der Ofenreise (HV-Ofen) aufgelistet, sowie die Streckgrenze (Rp), die Zugfestigkeit (Rm) und die Bruchdehnung (A). Die Zugproben wurden nach EN ISO 9693 wärmebehandelt, so daß ein Gefüge vorlag, wie es nach dem Keramikbrand existiert.

**Tabelle 2**

| Mechanische Eigenschaften | | | | | | |
|---|---|---|---|---|---|---|
| Leg. Nr. | HV-Guß | HV-hart | HV-Ofenr. | Rp (Mpa) | Rm(MPa) | A(%) |
| 1 | 220 | 235 | 230 | 560 | 720 | 12 |
| 2 | 210 | 250 | 220 | 480 | 650 | 17 |
| 3 | 230 | 258 | 248 | 520 | 718 | 12.7 |
| 4 | 217 | 220 | 211 | 480 | 672 | 14.5 |
| 5 | 216 | 248 | 238 | 490 | 780 | 18.1 |
| 6 | 202 | | 204 | 461 | 695 | 15.5 |
| 7 | 176 | | 184 | 455 | 648 | 18 |

Die erfindungsgemäßen Legierungen 6 und 7 weisen ein besonders angenehmes helles Oxid auf und besitzen mechanische Eigenschaften, die den Anforderungen an Typ 4-Legierungen genügen.

In den Tabellen 3, 4 und 5 sind die Ergebnisse der Korrosionsuntersuchungen zusammengestellt. Zur Bestimmung der Korrosionsbeständigkeit wurden Korrosionsversuche nach der Entwurfsfassung von ISO/DIS 1562 durchgeführt. Dazu werden Probekörper für 7 Tage in einer 0,1m Milchsäure-Kochsalzlösung bei 37 °C gelagert. Anschließend wird die Korrosionslösung mittels geeigneter Analyseverfahren (z.B. ICP) quantitativ auf die freigesetzten Korrosionsprodukte analysiert. Um Oberflächeneffekte und den Einfluß der Oxidation auszuschließen, werden die Prüfkörper nach einem zuvor simulierten Keramikbrand vor dem Einsetzen in die Korrosionslösung abgeschliffen. In den durchgeführten Untersuchungen wurden neben diesem "Standard-Korrosionstest" verschärfte Testbedingungen zusätzlich gewählt, um gerade den Einfluß der Oxidation auf das Korrosionsverhalten zu untersuchen. Dies ist notwendig, da davon auszugehen ist, daß unter realen Bedingungen nicht der gesamte Zahnersatz nach dem Keramikbrand so mechanisch nachbearbeitet werden kann, daß die vorausgehende oxidative Schädigung komplett entfernt werden kann. Zur Simulation dieser Bedingungen wurden ausgewählte Legierungen sandgestrahlt und oxidiert und ohne nachfolgende Entfernung der Oxidschicht in die Korrosionslösung gehängt. In der Tabelle 3 sind die jeweils analysierten Konzentrationen der gelösten Legierungsbestandteile für das Standardtestverfahren und in Tabelle 4 die Ergebnisse der verschärften Tests aufgeführt. In der letzten Spalte ist zusätzlich die Summe der Gesamtionenkonzentration aufgelistet, die das wesentliche Kriterium im ISO-Entwurf darstellt. Danach darf die Summe aller gelösten Ionen nicht den Grenzwert von 100µg/cm² überschreiten. Die Menge der Korrosionsprodukte wird dabei auf die Oberfläche der Probekörper bezogen. Die Korrosionsraten der Elemente, die unterhalb der jeweiligen Nachweisgrenze liegen, werden in dem Summenwert nicht berücksichtigt. Zusätzlich interessant ist natürlich auch das Langzeitkorrosionsverhalten der Legierungen. Daher wurden bei wenigen ausgewählten Legierungen auch Untersuchungen zum Korrosionsverhalten bis zu einer Gesamtzeitdauer von 12 Wochen durchgeführt. Um die Unterschiede stärker herauszuarbeiten, wurden auch hier die verschärften Testbedingungen, d.h. oxidierte Oberflächen getestet. Die Korrosionslösungen wurden dafür nach der 1, 4, 8, und 12 Wochen gewechselt und jeweils analysiert. Diese Ergebnisse sind in Tabelle 5 wiedergegeben.

**Tabelle 3**

| Konzentrationen der gelösten Elemente in den Korrosionslösungen in µg/cm² (Standard - Korrosionstest) | | | | | | | |
|---|---|---|---|---|---|---|---|
| Leg.-Nr. | Au | Pd | Ag | Sn | In | Sonstige | Gesamtkonzentration |
| 1 | <0.13 | <0.13 | - | | 0.36 | Ga:0.23 | 0.59 |
| 2 | <0.13 | <0.13 | <0.13 | <0.13 | - | Ga:0.21 | 0.21 |
| 4 | <0.13 | <0.13 | <0.13 | <0.13 | 0.90 | | 0.9 |
| 6 | <0.13 | <0.13 | | <0.13 | - | | 0 |

**Tabelle 4**

| Konzentrationen der gelösten Elemente in den Korrosionslösungen in µg/cm² (Verschärfte Testbedingungen) | | | | | | | |
|---|---|---|---|---|---|---|---|
| Leg.-Nr. | Au | Pd | Ag | Sn | In | Sonstige | Gesamtkonzentration |
| 1 | <0.13 | <0.13 | - | | 14,8 | Ga:5.5 | 20.3 |
| 2 | <0.13 | <0.13 | 0.44 | <0.13 | - | Ga:1.93 | 2.37 |
| 4 | <0.13 | <0.13 | 0.54 | | 9.3 | | 9.84 |
| 6 | <0.13 | <0.13 | 0.32 | <0.13 | | | 0.32 |
| 7 | <0.13 | <0.13 | 0.39 | <0.13 | - | | 0.39 |

**Tabelle 5**

| Gesamtkonzentrationen der gelösten Elemente in µg/cm² (Langzeittest - verschärfte Testbedingungen) | | | | |
|---|---|---|---|---|
| Leg.-Nr. | 1. Woche | 2.-4. Woche | 4.-8. Woche | 8.-12. Woche |
| 2 | 2.37 | 5.06 | 9.2 | 7.8 |
| 6 | 0.43 | 0.53 | 0.59 | 0.46 |

Die Ergebnisse belegen, daß die goldreduzierten Aufbrennlegierungen allesamt Korrosionswerte besitzen, die weit unter dem vorgeschriebenen Grenzwert von 100µg/cm² liegen. Es fällt jedoch auf, daß nur bei den erfindungsgemäßen Legierungen alle analysierten Elemente unterhalb oder knapp oberhalb der jeweiligen Nachweisgrenze liegen. Bei den verschärften Korrosions-Bedingungen wird dieser Unterschied zwischen den Legierungen, die den Stand der Technik darstellen, und den erfindungsgemäßen Legierungen noch deutlicher. Während die Legierungen nach dem Stand der Technik einen signifikanten Anstieg in den Korrosionsdaten aufweisen, zeigen die erfindungsgemäßen Legierungen auch unter diesen Bedingungen nur sehr geringe Korrosionsraten. Auch im Langzeittest zeigt sich bei der getesteten erfindungsgemäßen Legierung eine ca. um den Faktor 10 geringere Korrosionsrate als bei der Legierung nach dem Stand der Technik.

## Patentansprüche

1. Dentallegierung auf Basis von Gold-Palladium-Silber,
**dadurch gekennzeichnet,**
**daß** sie aus 46-49 Gew.% Gold, 28-35 Gew.% Palladium, 20-23 Gew.% Silber und 3,5-6 Gew.% Zinn besteht, wobei das Verhältnis von Palladium zu Zinn im Bereich 6 bis 8,5 liegt.

2. Dentallegierung nach Anspruch 1,
**dadurch gekennzeichnet,**
**daß** sie zusätzlich 0-1 Gew.% eines Kornfeiners aus der Gruppe Iridium, Ruthenium, Rhodium enthält.

3. Dentallegierung nach Anspruch 1 oder 2,
**dadurch gekennzeichnet,**
**daß** sie zusätzlich 0-2 Gew.% Platin enthält.

4. Verwendung von Legierungen gemäß den Ansprüchen 1 bis 3 zur Herstellung von mit Dentalkeramik verblendbarem Zahnersatz.

5. Festsitzender oder herausnehmbarer, mit Dentalkeramik verblendeter Zahnersatz,
**dadurch gekennzeichnet,**
**daß** das Metallgerüst aus einer Legierung gemäß den Ansprüchen 1 bis 3 gefertigt ist.

## Claims

1. A dental alloy based on gold-palladium-silver,
**characterised in that**
it consists of 46-49 wt.% of gold, 28-35 wt.% of palladium, 20-23 wt.% of silver and 3.5-6 wt.% of tin, wherein the ratio of palladium to tin is within the range from 6 to 8.5.

2. A dental alloy according to claim 1,
**characterised in that**
it additionally contains 0-1 wt.% of a grain-growth inhibitor from the group comprising iridium, ruthenium, rhodium.

3. A dental alloy according to claim 1 or claim 2,
**characterised in that**
it additionally contains 0-2 wt.% of platinum.

4. Use of alloys according to claims 1 to 3 for the production of a dental prosthesis which may be overlaid with dental ceramic.

5. A fixed or removable dental prosthesis overlaid with dental ceramic,
**characterised in that**
the metal skeleton is produced from an alloy according to claims 1 to 3.

## Revendications

1. Alliage dentaire à base d'or - palladium - argent,
**caractérisé en ce que**
il se compose de 46 à 49 % en poids d'or, 28 à 35 % en poids de palladium, 20 à 23 % en poids d'argent et 3,5 à 6 % en poids d'étain, le rapport entre le palladium et l'étain se situant dans la plage de 6 à 8,5.

2. Alliage dentaire selon la revendication 1,
**caractérisé en ce qu'**
il contient, en outre, 0 à 1 % en poids d'un affineur de granularité du groupe iridium, ruthénium, rhodium.

3. Alliage dentaire selon la revendication 1 ou 2,
**caractérisé en ce qu'**
il contient, en outre, 0 à 2 % en poids de platine ;

4. Utilisation d'alliages selon l'une quelconque des revendications 1 à 3 pour la fabrication d'une prothèse dentaire pouvant se combiner à de la céramique dentaire.

5. Prothèse dentaire fixe ou amovible combinée à de la céramique dentaire,
**caractérisée en ce que**
l'armature en métal est réalisée en un alliage selon l'une quelconque des revendications 1 à 3.
